# EUROPEAN PATENT APPLICATION

(11) **EP 1 574 190 A1**
(43) Date of publication of application: **14.09.2005**
(21) Application number: 05251341.3
(22) Date of filing: 07.03.2005
(51) Int. Cl.: A61F 5/44, A61F 13/64, A61F 5/00

(54) **Article for holding surgical dressings in place**

(30) Priority: 09.03.2004 GB 0405247
(71) Applicant: Ayrshire and Arran Health Board, Ayrshire KA6 6DX (GB)
(72) Inventor: Saw, T.K.K., Newton Mearns, Glasgow G77 6TJ (GB)
(74) Representative: Kennedy, David Anthony

(57) **Abstract**

An article for holding surgical dressings in place comprises a strip of material (1) which can be folded or bent to form a waistband (15). A section of material (8) can be detachably secured to the waistband (15) to form a back, front and gusset section of the article. The section of material can easily be detached from the waistband to allow access to the surgical dressings.

## Description

The present invention relates to an article for holding surgical dressings in place and, in particular, to apparel for holding wound dressings in place on uneven parts of the human body such as the groin region of a patient.

It is known in the art to use T bandages when dressing wounds in the perineal, scrotal, coccygeal, anal and vulval areas, following surgery. T bandages are shaped like the letter T and used principally for application to the groin or perineum. The bandages can be secured on the patient, and over wound dressings, in order to protect and hold the dressings in place.

These T bandages have a number of common disadvantages. It is difficult for the clinician or nursing staff to apply the bandage to the area, particularly if the patient is immobile or unconscious. The patient must either be lifted, or commonly rolled on one side to allow the bandage to be secured in place.

A further disadvantage is that the T bandage generally must be unfastened and removed from the patient to inspect the dressing underneath. This may be required frequently and soon after surgery. It is often necessary to change the bandage at regular intervals, and it will be appreciated that it is both difficult and time consuming for nursing staff to have to regularly remove and re-apply bandages to the affected area.

A further disadvantage is that as a result of the unrefined design of bandages, it can be difficult to obtain a secure yet comfortable fit on the patient on uneven areas of the body, such as the groin. The bandages currently used do not generally provide maximum conformity to this part of the body and may therefore provide insufficient support, or be cumbersome or uncomfortable to the patient.

Jock straps are sometimes used in place of these bandages as they provide more comfort to the patient. However these are also difficult to apply and are not intended to act as a dressing so provide poor ventilation and support and are not particularly hygienic.

It is an object of the present invention to provide an article for use after surgical procedures which addresses the problems described above.

It is a further object of the present invention to provide an article suitable for dressing wounds after surgery in the groin area.

According to a first aspect of the present invention there is provided an article for holding dressings in place in the groin area, the article comprising a strip of material which can be secured around the waist of a patient to form a waistband, and at least one section of material which can be detachably secured to the strip of material and is adapted to form a front and back section and gusset.

According to a second aspect of the present invention there is provided an article for holding dressings in place in the groin area, the article comprising a strip of material which can be secured around the waist of a patient to form a waistband, and at least one section of material which is completely detachable from the strip of material and is adapted to form a front and back section and gusset.

Preferably the section of material is an openwork material.

Preferably the article takes the form of a pair of underpants.

The article is preferably used to support and hold dressings in place in the perineal, scrotal, coccygeal, anal or vulval area of the patient.

Preferably the strip of material is manufactured from a pliable material which can be rolled or bent to form the waistband. The circumference of the waistband can be adjusted by altering the overlap of the ends of the strip of material when bent into the waistband shape.

Preferably the strip of material which forms the waistband includes one or more fastening means, provided to hold the strip of material around the waist of the patient. Preferably the fastening means are Velcro™ portions.

Preferably the openwork material is netting, mesh or the like. Most preferably the openwork material is washable.

Preferably the openwork material has a first and second end, wherein each of the first and second end has fastening means to allow the openwork material to be attached to the strip of material. Preferably the fastening means are Velcro™ strips.

Preferably when the strip of material is secured around the waist of the patient to form a waistband, the first end of the openwork material is attached to the strip of material at the patient's back, and the second end is attached to the strip of material at the patient's front. This allows the openwork material to form the front section, back section and gusset of the article. In this form, the article takes the form of a pair of underpants. The openwork material may have additional tightening means which allows the openwork material to be pulled in and tightened at the top of the patient's thighs for a secure fit.

The openwork material may optionally include means for exerting pressure to the wound, to allow bleeding control.

In one embodiment the openwork material may optionally include an opening to permit toilet access. In an alternative embodiment the gusset may be detachable.

The strip of material has an inner and outer surface. Preferably the inner surface is smooth.

Embodiments of the present invention will now be described by way of example only, and with reference to the following Figures, in which:
Figure 1 is a pictorial view of the inner surface of the unfastened waistband, in accordance with the present invention;
Figure 2 is a pictorial view of the outer surface of the unfastened waistband;
Figure 3 is a pictorial view of the open work material, which can be secured to the waistband shown in Figures 1 and 2;
Figure 4 is a pictorial view of the waistband of Figures 1 and 2 with the open work material of Figure 3 attached to form the article of the present invention;
Figure 5 is a pictorial view of the unfastened waistband of Figure 1 and 2 with the open work material of Figure 3 attached at one end, prior to use;
Figures 6 and 7 are pictorial views of the article being secured in position on a patient.

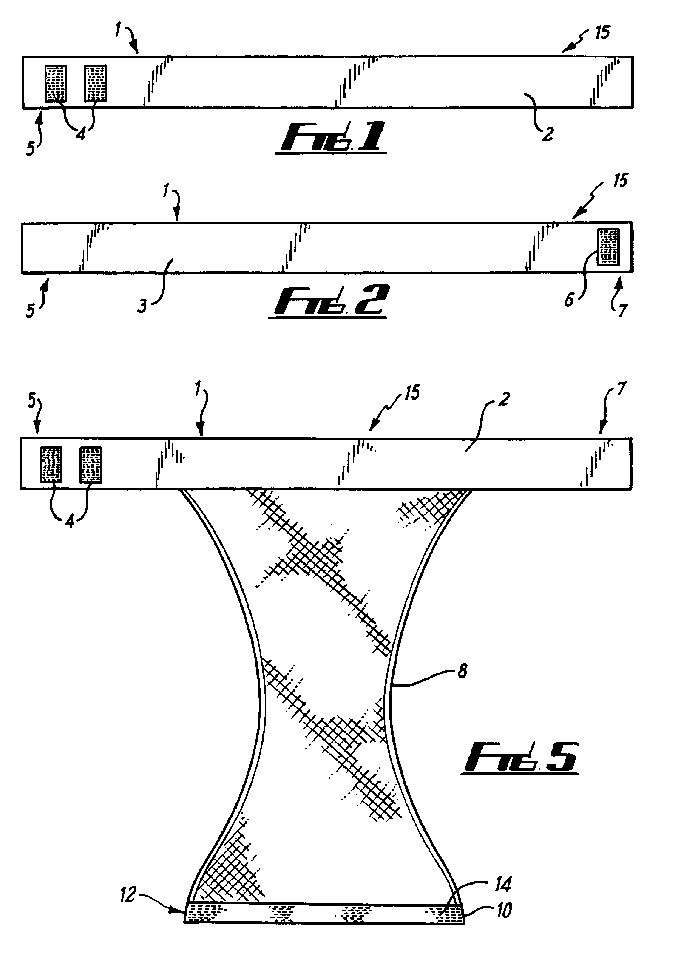

Referring initially to Figure 1 of the drawings, there is illustrated a strip of material 1 suitable for use as the waistband 15 of the article in accordance with the present invention. The strip of material 1 is of sufficient length to encircle the waist area of a patient. The strip of material 1 is manufactured from a flexible, durable material, which may be synthetic or non-synthetic and has an inner 2 and outer 3 surface. The outer surface 3 is typically of an uncut pile, as used in Velcro™ fastenings, whilst the inner surface 2, will typically has a smooth surface for a comfortable fit against the skin of the patient.

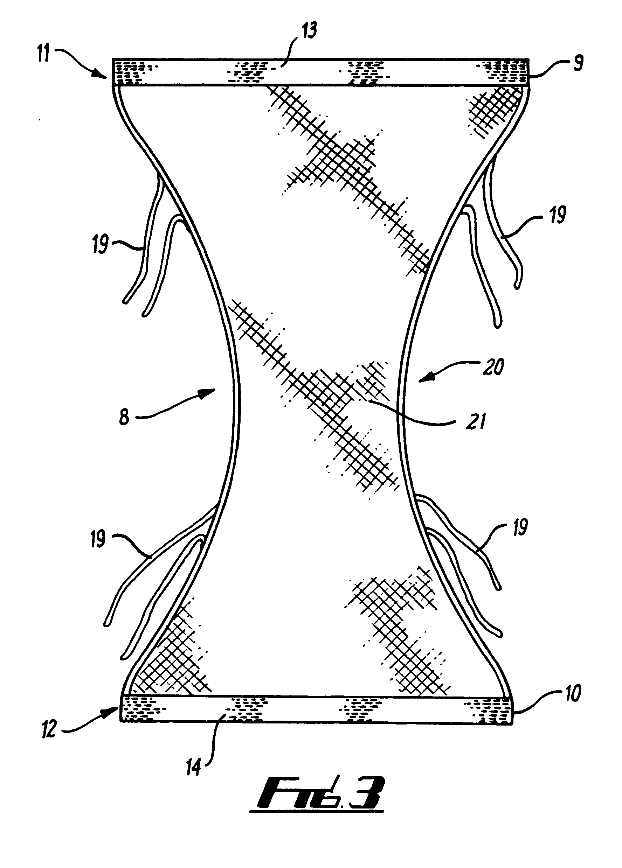

Figure 1 shows the inner surface 2 of the strip of material 1, whilst Figure 2 shows the outer surface 3 of the strip of material 1. The inner surface 2 has at least one portion of hooked material 4 of the type used in Velcro™ fastenings, located at one end 5. In the depicted embodiment, two Velcro™ portions 4 are shown, however it will be appreciated that this number is not limited. In an alternative embodiment, one hooked portion may be employed, whilst in other alternative embodiments a plurality of hooked portions may be present.

With reference to Figure 2, the outer surface 3 of the strip of material 1 contains a further hooked portion 6. Again, it will be appreciated that whilst in the depicted embodiment one hooked portion 6 is shown, in alternative embodiments two or more hooked portions may be present. The hooked portion 6 is located at the opposite end 7 of the strip of material 1 to the hooked portions 4 located on the inner surface 2.

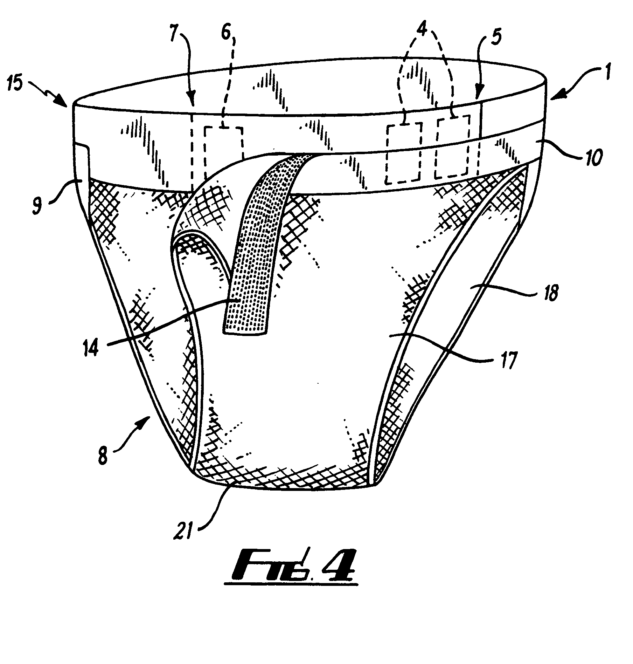

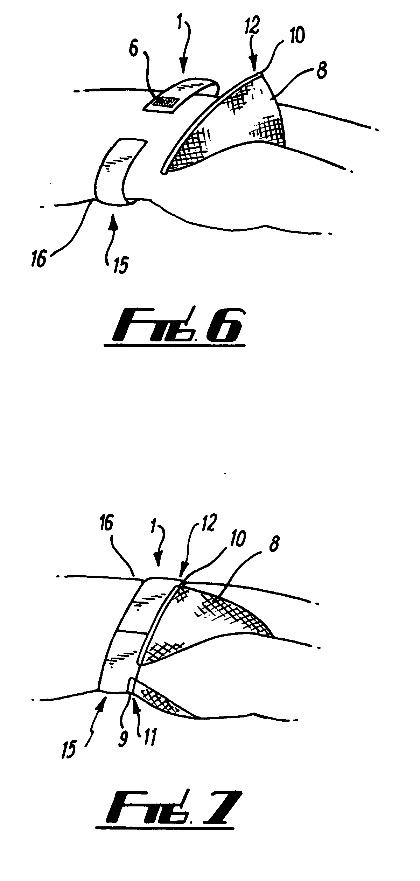

The strip of material 1 is preferably formed from a material which is rollable and bendable into a circular waistband 15. When folded into a waistband 15, as shown in Figure 4, ends 5 and 7 are held in an overlap by the Velcro™ hooked portions 4 on the inner surface 2 of the strip of material 1, and also the Velcro™ hooked portion 6 on the outer surface 3 of the strip of material 1. The Velcro™ portions are shown in Figure 4 as broken lines as they are not visible when the waistband 15 is fastened.

The provision of the Velcro™ hooked portions 4 and 6 on both the inner 2 and outer 3 surfaces of the strip of material 1 advantageously means that the overlap between ends 5 and 7 can be increased or decreased when the strip of material 1 is folded, in order to change the circumference of the waistband 15. This is an important aspect of the present invention as the size of the waistband 15 can be adjusted to ensure a snug and comfortable fit on the patient. As the article can be adjusted in size to provide a better fit to the patient, this provides better support to the dressings underneath.

Reference is now made to Figure 3, which shows the section of material 8, which forms the main body of the article and which can be attached to the waistband 15 shown in Figures 1 and 2. The section of material is an openwork material in the preferred and depicted embodiment. The openwork material 8 has borders 9,10 at first and second end 11, 12. The borders 9,10 have Velcro™ hooked strips 13,14. Advantageously, the Velcro™ hooked strips 13,14 can be detachably secured to the outer surface 3 of the waistband 15 shown in Figures 1 and 2. In this manner the waistband 15 and openwork material 8 can be attached to form pant-like article as shown in Figure 3.

It will be appreciated that when attached to the waistband 15 the openwork material 8 forms the main body of the article and, similar to a normal pair of underpants has what can be termed as a front 17 and back 18 section and gusset 19 area. It should be appreciated that the openwork material 8 may be of a one-piece construction or alternatively comprise more than one piece of material. For example in an alternative embodiment the main body of the article could be formed from a first and second section of material which can be in some manner connected together when in use to form the front and back section and gusset of the article, as opposed to the one-piece section of openwork material 8 shown in the Figures. In such an embodiment both the first and second section could be independently attached to the waistband 15 and then connected together to form the main body around the groin area of the patient, using any suitable connection means known in the art for example studs.

In the preferred and depicted embodiment the openwork material 8 is of a one-piece construction and therefore reference to front and back sections are intended to describe the area against which the openwork material 8 sits on the patient's body. The terms front and back section and gusset are not intended to limit the section of material to any particular shape or to a three piece construction but rather to define more clearly the area of the patient's body against which the main body of the article formed by the section of material sit. To further ensure a snug and comfortable fit on the patient the openwork material may optionally have tightening means in the form of velcro straps or ties 19 which allow the openwork material to be pulled in and tightened at and around the patient's thighs for a secure fit.

As can be seen in Figure 3, the openwork material 8 has a substantially hourglass shape. Using the analogy of an hourglass the neck area 20 forms the gusset 19 or crotch area of the article.

The openwork material 8 is typically in the form of an elasticated netting or mesh. The material is washable and quick drying. The netting or mesh is of sufficient strength and robustness to maintain its shape and hold dressings applied to the groin area of the patient in position. However the netting or mesh is also pliable and comfortable to wear. The advantage of using a netting or mesh material, lies in the fact that the dressings can be viewed without having to remove the article from the patient. In addition, the use of an openwork material provides ventilation to the area which has been dressed and is hygienic in use. Advantageously the openwork material is washable and quick drying. The openwork material 8 may also optionally have a detachable gusset or, an openable area in the gusset to allow the patient to go to the toilet without having to completely remove the article.

Reference is now made to Figure 4 of the drawings. The openwork material 8 is initially attached to the strip of material 1 by the Velcro™ hooked strip 13 at first end 11 of the openwork material. Velcro™ hooked strip 13 (not shown in Figure 4) is attached to the outer surface 3 of the strip of material 1. When the article is in the form shown in Figure 4 it can be slid or passed under the patient's waist, on the bed or operating table, with minimal disturbance to the patient.

The strip of material 1 can then be folded around the patient's waist 16 (as shown in Figures 5 and 6) and secured using the Velcro™ hooked portions 4,6 as described above, to form waistband 15. In position, the Velcro™ strip 13 at first end 11 is attached to the outer surface 3 of waistband 15 at the back of the patient.

Once the waistband 15 has been fastened around the patient's waist 16, the open work material 8 can be passed between the patient's legs and Velcro™ hooked strip 14 at second end 12 can be secured to the front of the outer surface 3 of the waistband 15, as shown in Figure 7. The tightening means in the form of velcro straps or ties 19 (shown in Figure 3) can then be adjusted to pull in and tighten the openwork material around the patient's thighs for a secure fit

Although the example described herein provides one method of positioning the article on the patient, it will be appreciated that other methods are possible and may indeed be preferred. For example, the waistband 15, may be secured in position around the patient's waist 16 prior to attaching the first 11 and second 12 ends of the openwork material. Alternatively the openwork material 8 may be attached to the strip of material 1 using Velcro™ strip 14 at second end 12, and then placed under the patient, before the openwork material is passed between the patient's legs and secured.

In the abovedescribed embodiment the section of material 8 can be completely detached and removed from the strip of material 1. Alternatively the section of material 8 may be provided partially attached to the strip of material 1. For example one of the ends 11,12 of the section of material may be permanently secured, fixed or attached to the strip of material 1 which forms the waistband 15. In this embodiment only the end 11,12 opposite the end which is permanantly secured, fixed or attached will have a Velcro™ hooked strip to allow the section of material to be detachably secured to the waistband 15 when in position on the patient.

The article may also be reinforced, manufactured from a robust supportive material, or have other means to provide pressure to the area which is dressed.

A principal advantage of the article herein described is that it can be easily positioned and secured, even whilst the patient is prostrate, minimising the need to lift or roll the patient onto one side, as required when T bandages or jockstraps are used.

The article can be used on either males or females, and is suitable for use on scrotal, perineal, anal, coccygeal and vulval areas.

A further advantage of at least one aspect of the present invention, is that the openwork material can easily be detached and re-attached to the waistband. This allows the main body of the article to removed for washing and replaced, if soiled.

A particular advantage is that the ease of detachment of the openwork material ensures that dressings in the groin area can be accessed and checked quickly and simply by detaching one end of the openwork material and pulling it down.

A yet further advantage of the present invention lies in the fact that as an openwork material is used it is possible for the dressings to be checked, without having to move the patient or indeed remove the article from the patient's body.

Modifications may be made to the invention described hereinbefore, without departing from the scope thereof.

## Claims

1. An article for holding dressings in place in the groin area of a patient, **characterised in that** the article comprises a strip of material (1) which can be secured around the waist of a patient to form a waistband (15), and at least one section of material (8) which can be detachably secured to the strip of material (1) adapted to form a front and back section and a gusset of the article.

2. An article as claimed in Claim 1 where the at least one section of material (8) can be completely detached from the strip of material (1).

3. An article as claimed in Claims 1 and 2 wherein the section of material (8) is an openwork material.

4. An article as claimed in Claims 1 to 3 which takes the form of a pair of underpants.

5. An article as claimed in any of the preceding Claims used to support and hold dressings in place in the perineal, scrotal, coccygeal, anal or vulval area of the patient.

6. An article as claimed in any of the preceding Claims, where the strip of material (1) is manufactured from a pliable material which can be rolled or bent to form the waistband (15).

7. An article as claimed in any of the preceding Claims where the strip of material (1) is configured such that the circumference of the waistband (15) is adjusted by changing the amount of overlap of the ends (5,7) of the strip of material (1) when it is rolled or bent to form the waistband (15).

8. An article as claimed in any of the preceding Claims where the strip of material (1) which forms the waistband (15) includes one or more fastening means (4, 6), provided to hold the strip of material (1) around the waist of the patient.

9. An article as claimed in Claim 8 where the fastening means (4,6) are Velcro™.

10. An article as claimed in Claim 3 where the openwork material is a netting or mesh.

11. An article as claimed in any of the preceding Claims where the section of material (8) is washable.

12. An article as claimed in any of the preceding Claims where the section of material (8) has a first and second end (11,12), wherein each of the first and second ends (11,12) has fastening means (13,14) to allow the section of material (8) to be attached to the strip of material (1).

13. An article as claimed in Claim 12 where the fastening means are Velcro™ strips.

14. An article as claimed in any of the preceding Claims where the section of material (8) has additional tightening means (19) which allows the section of material (8) to be adjusted at the top of the patient's thighs for a secure fit.

15. An article as claimed in any of the preceding Claims where the section of material (8) includes means for exerting pressure to the wound, to allow bleeding control.

16. An article as claimed in any of the preceding claims where the inner surface (2) of the strip of material (1) is smooth.

17. An article as claimed in any of the preceding Claims having an opening to permit toilet access.
